# EUROPEAN PATENT SPECIFICATION

(11) **EP 1 428 870 B1**
(45) Date of publication and mention of the grant of the patent: **19.11.2008**
(21) Application number: 02751195.5
(22) Date of filing: 30.07.2002
(51) Int. Cl.: C12N 1/20, C12N 1/36

(54) **REDUCTION IN THE VIRULENCE OF MYCOBACTERIUM TUBERCULOSIS AND PROTECTION AGAINST TUBERCULOSIS BY MEANS OF PHOP GENE INACTIVATION**
VERRINGERUNG DER VIRULENZ VON MYCOBACTERIUM TUBERCULOSIS UND TUBERKULOSESCHUTZ MITTELS INAKTIVIERUNG DES PHOP-GENS
REDUCTION DE LA VIRULENCE DE MYCOBACTERIUM TUBERCULOSIS ET PROTECTION CONTRE LA TUBERCULOSE PAR L'INACTIVATION DU GENE PHOP

(30) Priority: 31.07.2001 ES 200101824
(43) Date of publication of application: 16.06.2004
(73) Proprietor: Universidad de Zaragoza, E-50005 Zaragoza (ES)
(72) Inventor: MARTIN MONTANES, Carlos, E-50005 Zaragoza (ES); GICQUEL, Brigitte, 75724 Paris Cedex 15 (FR); PEREZ HERRAN, Ester, E-50005 Zaragoza (ES)
(74) Representative: Pons Arino, Angel
(86) International application number: PCT/ES2002/000381
(87) International publication number: WO 2003/012075

(56) References cited:
- WO-A-90/11687
- WO-A-95/02048
- WO-A-97/20033
- PEREZ E. ET AL.: 'An essential rol for pho P in mycobacterium tuberculosis virulence' MOLECULAR MICROBIOLOGY vol. 41, no. 1, 2001, pages 179 - 187, XP002978899
- ZAHRT T.C. ET AL.: 'An essential two component signal transduction system in mycobacterium tuberculosis' JOURNAL OF BACTERIOLOGY vol. 182, no. 13, 2000, pages 3832 - 3838, XP002978898
- ZAHRT T.C. ET AL.: 'Elements of signal transduction in mycobacterium tuberculosis: In vitro phosphorylation and in vivo expression of the response regulator MtrA' JOURNAL OF BACTERIOLOGY vol. 178, no. 11, 1996, pages 3314 - 3321, XP002195607
- ZAHRT T.C. ET AL.: 'Mycobacterium tuberculosis signal transduction system required for persistent infections' PROC. NATL. ACAD. SCI. USA vol. 98, no. 22, 2001, pages 12706 - 12711, XP002978900

## Description

### FIELD OF APPLICATION

This invention falls within the field of microbiology.

### BACKGROUND OF THE INVENTION:

The recent development of genetic tools to handle the tuberculosis bacillus allows us to build mutants in specific genes.
In other intracellular pathogen microorganisms such as *Salmonella,* it has been described that the *phoP* gene is involved in virulence. The inactivation of this gene has allowed us to build attenuated mutants that have been studied as vaccines against Salmonellosis in humans.
Our previous results (Zaragoza University) in strains of the *M*. *tuberculosis* complex, that were isolated in humans, indicated that strains that are probably more virulent, present alterations in the control of the gene recorded in the *M. tuberculosis* genome, as *phoP*.
In order to study the involvement of *phoP* in the *M*. *tuberculosis* virulence, the gene was inactivated in clinical isolation, and we studied its capacity to multiply and survive in macrophages and in mice.

### DESCRIPTION OF THE INVENTION

We built vectors, where we inactivated the *M. tuberculosis phoP* gene, by introducing a kanamycin resistant .gene. The vectors were transformed into a clinical isolate of *M. tuberculosis,* obtaining a strain with inactivated *phoP* gene. The details of these constructions appear in the laboratory workbooks for the years 1998-1999 of the Mycobacterial Genetics Group of the Zaragoza University and of the Unite de Genetique Mycocterienne of the Pasteur Institute in Paris.

The *M. tuberculosis phoP* mutant was placed on November 27, 2000 in the culture collection of the Pasteur Institute of Paris (I-2622), 28, rue du Dr. Roux, 75724 Paris Cedex 15, France.

When we studied *M*. *tuberculosis*, and compared it with the mutant *M. tuberculosis phoP* strain for 7 days in macrophage cultures of mice bone marrow, we observed that the mutant is not capable of replicating itself in a mammal macrophage. When we continued the mutant culture for 14 days, we observed that it was capable of surviving.

After infecting a mouse with the mutant strain, we observed that the bacillus did not multiply either after 3 or after 6 weeks, but the number of bacteria was not reduced, with respect to those of the initial inoculate, in the spleen, the liver or the lungs.

The results of the experiments with the *M*. *tuberculosis phoP* strain in macrophages and in mice, appear in the laboratory workbooks of the Mycobacterial Genetics Group of the Zaragoza University, and of the Unite de Genetique Mycocterienne of the Pasteur Institute in Paris for the year 2000.

To these results, we should add the mice protection studies. The *M*. *tuberculosis phoP* mutant was inoculated subcutaneously. Eight weeks later, the mice were infected intravenously with *M*. *tuberculosis* H37Rv. The bacterial recount after 4 weeks of infection, clearly showed that the *phoP* mutant protects against the multiplication of *M*. *tuberculosis*, with effectiveness comparable to the BCG vaccine control. The results of the experiments appear in the workbooks of the Unite de Genetique Mycocterienne of the Pasteur Institute in Paris for the years 2001-2002.

### DESCRIPTION OF THE FIGURES

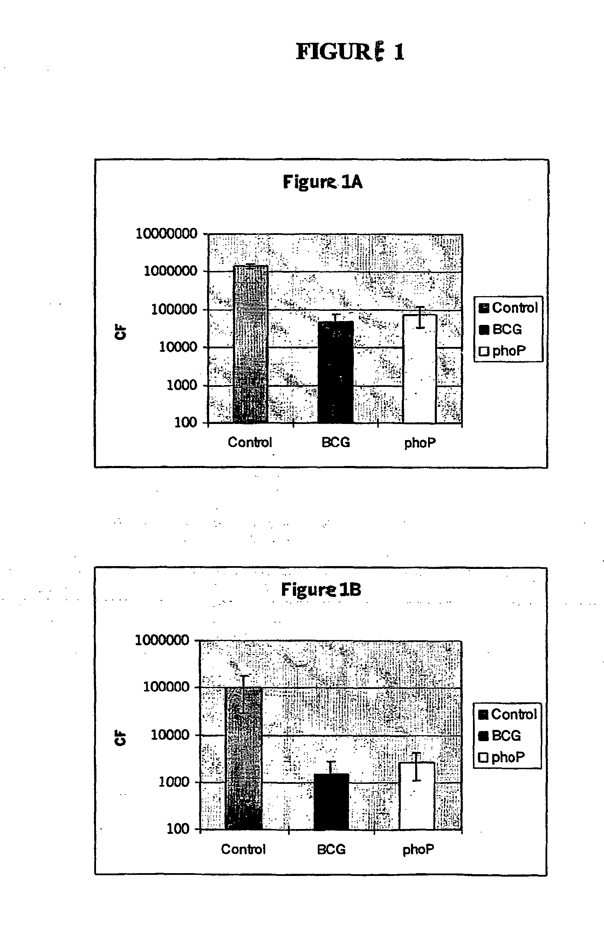

FIGURE 1 - Protection of the *M*. *tuberculosis phoP* mutant against *M*. *tuberculosis* H37Rv infection.
Balb/c mice were inoculated subcutaneously with 10⁷ colony-forming units (CFU's) either of the control vaccine against BCG tuberculosis or of the vaccine candidate of the *M. tuberculosis phoP* mutant.

After eight weeks, the mice were infected intravenously with *M*. *tuberculosis* H37Rv (10⁷ CFU's).

Four weeks after the infection, the animals were put down and we counted the CFU's in the spleen (Figure 1A) and the lung (Figure 1B). Each value represents the geometric mean +/- SEM. *[sic]*

## Claims

1. A vaccine suitable for protecting against *Mycobacterium tuberculosis* infection in mammals which comprises a *Mycobacterium* strain **characterized in that** its virulence is attenuated by inactivation of the *phoP* gene.

2. The vaccine of claim 1, wherein the mammal is a human.

3. The vaccine of anyone of claims 1-2 for its use in the treatment of tuberculosis disease.

4. Use of a *Mycobacterium* strain **characterized in that** its virulence is attenuated by inactivation of the *phoP* gene for the manufacture of the vaccine of anyone of claims 1-2.

## Patentansprüche

1. Ein Impfstoff zum Schutz gegen eine Infektion mit *Mycobacterium tuberculosis* von Säugetieren, die einen *Mycobacterium-*Stamm umfasst, **dadurch gekennzeichnet, dass** dessen Virulenz durch eine Inaktivierung des phoP-Gens abgeschwächt ist.

2. Der Impfstoff nach Anspruch 1, wobei es sich bei dem Säugetier um einen Menschen handelt.

3. Der Impfstoff nach irgendeinem der Ansprüche 1-2 für dessen Verwendung bei der Behandlung der Tuberkulose-Erkrankung.

4. Die Verwendung eines *Mycobacterium-*Stammes, **dadurch gekennzeichnet, dass** dessen Virulenz durch die Inaktivierung des phoP-Gens zur Herstellung des Impfstoffes nach irgendeinem der Ansprüche 1-2 abgeschwächt ist.

## Revendications

1. Un vaccin destiné à protéger contre l'infection à *Mycobacterium tuberculosis* chez les mammifères qui comprend une souche de *Mycobacterium* **caractérisée en ce que** sa virulence est atténuée par l'inactivation du gène *pho*P.

2. Le vaccin de la revendication 1, où le mammifère est un humain.

3. Le vaccin de n'importe laquelle des revendications 1-2 pour son utilisation dans le traitement de la tuberculose.

4. Utilisation d'une souche de *Mycobacterium* **caractérisée en ce que** sa virulence est atténuée par l'inactivation du gène *pho*P pour la fabrication du vaccin de n'importe laquelle des revendications 1-2.
